# EUROPEAN PATENT SPECIFICATION

(11) **EP 4 480 324 B1**
(45) Date of publication and mention of the grant of the patent: **24.12.2025**
(21) Application number: 23845925.9
(22) Date of filing: 18.04.2023
(51) Int. Cl.: A23L 29/00, A23L 29/20, A23L 29/262, A23L 29/30, A23L 5/00, A61J 3/07, A61K 9/70, A61K 47/26, A61K 47/32, A61K 47/34, A61K 47/36, A61K 47/38, A61J 3/00

(54) **EDIBLE FILM**
ESSBARE FOLIE
FILM COMESTIBLE

(30) Priority: 26.07.2022 JP 2022119027
(43) Date of publication of application: 25.12.2024
(73) Proprietor: NISSHA CO., LTD., Kyoto-shi, Kyoto 604-8551 (JP)
(72) Inventor: CHIYAMA, Masateru, Kyoto-shi, Kyoto 604-8551 (JP)
(74) Representative: Isarpatent
(86) International application number: PCT/JP2023/015521
(87) International publication number: WO 2024/024185

(56) References cited:
- WO-A1-2008/149440
- WO-A1-2017/135195
- CN-A- 104 013 602
- CN-A- 104 223 078
- JP-A- 2000 116 339
- JP-A- 2008 538 782
- JP-A- 2010 265 217
- JP-A- 2012 121 873
- JP-A- 2017 520 535
- JP-A- 2019 156 721
- JP-A- 2019 156 721
- JP-A- 2021 054 738
- JP-A- H10 215 792
- JP-B1- 7 168 133
- ANONYMOUS: "Lusefi OD film 2.5mg ", TAISHO PHARMA CO LTD, 1 March 2022 (2022-03-01), pages 1 - 7, XP093133465, Retrieved from the Internet <URL:https://medical.taisho.co.jp/di/guide/400059_39690B1F1024_1_01G.pdf> [retrieved on 20240221]
- ANONYMOUS: "Makanai Cosme “UV protection supplement that you can take before going out", PR TIMES, 11 March 2019 (2019-03-11), pages 1 - 5, XP093133471, Retrieved from the Internet <URL:https://prtimes.jp/main/html/rd/p/000000143.000010257.html> [retrieved on 20240221]

## Description

### TECHNICAL FIELD

The present invention relates to an edible film.

### BACKGROUND

Known edible films include film pharmaceuticals and film confectionery. For example, Patent Literature 1 describes an edible film formed from an orally soluble base material containing medicines and additives. Patent Literature 2 relates to a tea polyphenol oral instant film which has good taste and can rapidly disintegrate in the oral cavity, and whose preparation process is simple and suitable for mass production.

### CITATION LIST

### PATENT LITERATURE

Patent Literature 1: Japanese Patent No. 6349391
Patent Literature 2: Chinese application No. CN104223078A

### BRIEF SUMMARY

### TECHNICAL PROBLEM

Known edible films as described above are manufactured with a coating method. The coating method includes dissolving an edible base material and other ingredients in a liquid and applying the solution onto a carrier film to form a thin coating. The coating is then dried to be an edible film.

However, when the solution used in manufacturing an edible film with the coating method has a high viscosity, the solution may flow less smoothly and cause difficulty in achieving uniform coating.

When the solution has a low viscosity, convection currents (Benard convection) can occur during the drying process and may cause nonuniform distribution of the ingredients contained in the edible film.

In response to the above issue, one or more aspects of the present invention are directed to an edible film that is easy to manufacture with a coating method and contains ingredients in a uniformly distributed manner.

### SOLUTION TO PROBLEM

The present invention is directed to an edible film as defined in appended claim 1. An edible film according to the present invention includes a base material, and at least one ingredient including microcrystalline cellulose. A content of the microcrystalline cellulose is 5 to 50 wt%.

A solution with the above composition is less viscous and more flowable during the coating process in which the solution is sheared. Thus, the coating process in manufacturing the edible film is easy.

When the solution is not sheared after coating, the solution is more viscous and less flowable. This reduces convection during the drying process and allows uniform distribution of the ingredients in the edible film.

In the edible film according to the present invention, the at least one ingredient further includes maltitol.

The above composition reduces aggregation of the microcrystalline cellulose and reduces nonuniform distribution of the ingredients in the edible film.

In the edible film according to the present invention, the base material is one of polyvinyl alcohol, polyethylene oxide, or polyvinylpyrrolidone. When the base material is polyvinyl alcohol, a weight ratio of a content of the microcrystalline cellulose to a content of the maltitol is 0.5 to 3.3.

Polyvinyl alcohol used as the base material further reduces aggregation of the microcrystalline cellulose, more effectively reducing nonuniform distribution of the ingredients in the edible film.

In the edible film according to the present invention, when the base material is polyethylene oxide, a weight ratio of a content of the microcrystalline cellulose to a content of the maltitol is 0.5 to 7.5.

The above composition containing polyethylene oxide as a base material further reduces aggregation of the microcrystalline cellulose, more effectively reducing nonuniform distribution of the ingredients in the edible film.

In the edible film according to the present invention, when the base material is polyvinylpyrrolidone, a weight ratio of a content of the microcrystalline cellulose to a content of the maltitol is 0.67 to 7.5.

The above composition containing polyvinylpyrrolidone as the base material further reduces aggregation of the microcrystalline cellulose, thus reducing nonuniform distribution of the ingredients in the edible film.

Also disclosed herein but not forming part of the present invention is an edible film that includes a dried solution containing a base material and an ingredient. The ingredient includes microcrystalline cellulose, and η₀/η₁₀₀ is 1.5 to 10, where η₀/η₁₀₀ is a ratio of a zero shear viscosity η₀ of the solution at a temperature of 25 °C to a viscosity η₁₀₀ of the solution measured at a temperature of 25 °C and a shear rate of 100/s.

Also disclosed herein but not forming part of the present invention is a method for manufacturing an edible film that includes preparing a solution containing a base material and an ingredient with η₀/η₁₀₀ of 1.5 to 10, where η₀/η₁₀₀ is a ratio of a zero shear viscosity η₀ of the solution at a temperature of 25 °C to a viscosity η₁₀₀ of the solution measured at a temperature of 25 °C and a shear rate of 100/s, coating a carrier film with the solution, drying the solution after the coating, and cutting the dried solution into pieces.

The solution is less viscous and more flowable during the coating process in which the solution is sheared. Thus, the coating process in manufacturing the edible film is easy.

When the solution is not sheared after coating, the solution is more viscous and less flowable. This reduces convection during the drying process and allows uniform distribution of the ingredients in the edible film.

### ADVANTAGEOUS EFFECTS

The edible film according to the above aspects of the present invention is easy to manufacture with a coating method and contains the ingredients in a uniformly distributed manner.

### BRIEF DESCRIPTION OF DRAWINGS

FIG. 1 is a flowchart showing an example method for manufacturing an edible film according to an embodiment of the present disclosure.
FIG. 2 is a schematic cross-sectional view of an example mixer.
FIG. 3 is a schematic cross-sectional view of an example coating device.
FIG. 4 is a schematic plan view of the edible film in a process of being cut into pieces.
FIG. 5 is a graph showing the relationship between shear rate and viscosity based on measurements of a solution containing polyvinyl alcohol as a base material.
FIG. 6 is a graph showing the relationship between shear rate and viscosity based on measurements of a solution containing polyethylene oxide as a base material.
FIG. 7 is a graph showing the relationship between shear rate and viscosity based on measurements of a solution containing polyvinylpyrrolidone as a base material.
FIG. 8A is a micrograph of an edible film containing polyvinyl alcohol as a base material and not containing maltitol as an ingredient, and FIG. 8B is a micrograph of an edible film containing polyvinyl alcohol as a base material and containing maltitol as an ingredient.
FIG. 9A is a micrograph of an edible film containing polyethylene oxide as a base material and not containing maltitol as an ingredient, and FIG. 9B is a micrograph of an edible film containing polyethylene oxide as a base material and containing maltitol as an ingredient.
FIG. 10A is a micrograph of an edible film containing polyvinylpyrrolidone as a base material and not containing maltitol as an ingredient, and FIG. 10B is a micrograph of an edible film containing polyvinylpyrrolidone as a base material and containing maltitol as an ingredient.

### DETAILED DESCRIPTION

One or more embodiments of the present invention will now be described with reference to the drawings.

An edible film 10 according to an embodiment of the present invention is, for example, a rectangular thin film in a plan view with a thickness of 10 to 300 µm, or more specifically, 30 to 200 µm. The edible film 10 is formed mainly from a base material and ingredients blended with the base material. Examples of the edible film 10 include those that dissolve or disintegrate in the oral cavity to release ingredients and those that are swallowable and digestible in the stomach or intestines to allow ingredients to be absorbed. Ingredients blended with the base material include microcrystalline cellulose and maltitol. In addition to these ingredients, the edible film 10 may contain various other materials as ingredients as appropriate for the purpose. Examples of the other materials include medicinal ingredients to provide medicinal efficacy with the edible film 10, flavor ingredients to add flavors to the edible film 10, coloring ingredients to color the edible film 10, nutritional ingredients (including supplements) to add nutrition to the edible film 10, aromatic ingredients to add aroma to the edible film 10, and additives to adjust the physical properties of the edible film 10.

Examples of additives include binders, excipients, disintegrants, flavoring agents, wetting agents, coloring agents, and emulsifiers.

The base material is the main material for forming the edible film 10.

The base material may be, for example, an edible organic compound or an edible inorganic compound. Examples of edible organic compounds include edible carbohydrates, edible proteins, and edible fats. Examples of edible carbohydrates include edible disaccharides, edible polysaccharides, edible sugar alcohols, and edible dietary fiber. Examples of edible polysaccharides include alginic acid, sodium alginate, pregelatinized starch, carrageenan, agar, xanthan gum, potato starch, cellulose, and pullulan. Examples of edible dietary fiber include pectin and cellulose. Examples of edible disaccharides include sucrose. Examples of edible sugar alcohols include sorbitol. Examples of edible proteins include gelatin. Examples of edible organic compounds other than carbohydrates, proteins, and fats include polyvinyl alcohol (PVA), polyethylene oxide (PEO), polyvinylpyrrolidone (PVP or povidone), and macrogol (polyethylene glycol). Examples of their edible derivatives include, for example, sugar derivatives, cellulose derivatives, PVA derivatives, and sorbitol derivatives. Examples of sugar derivatives include, for example, sucrose fatty acid esters. Examples of cellulose derivatives include ethylcellulose, carmellose (CMC), carmellose sodium, and hypromellose (HPMC). Derivatives of sorbitol include sorbitan and sorbitan fatty acid esters (polysorbate). Among these, PVA, PEO, or PVP are used in the edible film according to the present invention. PVA, PEO, or PVP are used as a base material to form strong and flexible edible films.

Microcrystalline cellulose is used to improve the thixotropy of a solution 32 that is a raw material of the edible film 10. Thixotropy is a property of the solution 32 to be less viscous when the solution 32 is sheared and to be more viscous when the solution 32 is not sheared. When having high thixotropy, the solution 32 is sheared and is less viscous and more flowable during the coating process. The solution 32 is thus applied easily in the coating process. After the coating process, the solution 32 is not sheared and is more viscous and less flowable. This reduces convection (Benard convection) particularly in the process of drying the solution 32, reducing nonuniform distribution of ingredients.

Thixotropy is higher when the microcrystalline cellulose content is higher, but the microcrystalline cellulose content is not to be too high when an ingredient other than microcrystalline cellulose is to be contained at a high level. In the edible film according to the present invention, the microcrystalline cellulose content is 5 to 50 wt%, specifically 7.5 to 45 wt%, or more specifically 10 to 40 wt%. The edible film 10 can have high thixotropy with a microcrystalline cellulose content of 5 wt% or more, 7.5 wt% or more, or more specifically, 10 wt% or more. The microcrystalline cellulose content of 50 wt% or less, 45 wt% or less, or 40 wt% or less is low enough to allow other ingredients to be sufficiently contained. In this embodiment, the microcrystalline cellulose content refers to the amount of microcrystalline cellulose contained in the edible film 10 in a solid state obtained by drying the solution 32.

In the edible film according to the present invention, maltitol is used to reduce aggregation of microcrystalline cellulose. Although microcrystalline cellulose reduces nonuniform distribution of ingredients resulting from convection by alleviating convection in the solution 32 in the drying process, microcrystalline cellulose can aggregate in the edible film 10 and cause nonuniform distribution of ingredients including the microcrystalline cellulose. However, maltitol blended with the base material can reduce aggregation of microcrystalline cellulose.

The maltitol content may be, for example, 1 to 35 wt%, specifically 2 to 25 wt%, or more specifically 3 to 15 wt%, although being largely affected by its ratio to the microcrystalline cellulose content. With a maltitol content of 1 wt% or more, 2 wt% or more, or more specifically 3 wt% or more, the ratio of maltitol to microcrystalline cellulose is likely to be higher, expectedly reducing aggregation of microcrystalline cellulose. With a maltitol content of 35 wt% or less, 25 wt% or less, or more specifically 15 wt% or less, the drying speed of the solution 32 is less likely to be slow and the edible film 10 is less likely to be sticky. In this embodiment, the maltitol content refers to the amount of maltitol contained in the edible film 10 in a solid state obtained by drying the solution 32.

A binder increases the strength of the edible film 10. Examples of binder materials include amylopectin, sodium alginate, pregelatinized starch, carmellose, carmellose sodium, agar, glycerin, crystalline cellulose, polymer polyvinylpyrrolidone, wheat starch, rice starch, sucrose fatty acid esters, purified gelatin, purified shellac, sucrose, gelatin, soy lecithin, low substituted hydroxypropyl cellulose, dextrin, concentrated glycerin, crystalline cellulose, hydroxyethyl cellulose, hypromellose, pullulan, pectin, polysorbate, macrogol, D-mannitol, and methylcellulose.

An excipient increases the volume of the edible film 10 to a size that is easy to handle. Examples of excipient materials include alginic acid, sodium alginate, pregelatinized starch, ethylcellulose, carrageenan, carmellose, carmellose sodium, agar, glycerin, croscarmellose sodium, crospovidone, magnesium silicate, crystalline cellulose, wheat flour, wheat starch, rice flour, rice starch, titanium dioxide, sucrose fatty acid esters, sucrose, gelatin, skimmed milk powder, talc, dextran, dextrin, potato starch, hypromellose, pullulan, pectin, polysorbate, macrogol, maltose, and methylcellulose.

A disintegrant provides disintegratability to the edible film 10. Examples of disintegrant materials include alginic acid, pregelatinized starch, carmellose, carmellose sodium, agar, croscarmellose sodium, crospovidone, crystalline cellulose, wheat starch, rice starch, sucrose fatty acid esters, gelatin, dextrin, corn starch, potato starch, hydroxypropyl cellulose, hypromellose, polysorbate, macrogol, magnesium aluminometasilicate, methylcellulose, and sodium lauryl sulfate.

A flavoring agent adjusts the flavor of the edible film 10. Examples of flavoring agent materials include aspartame, DL-alanine, erythritol, reduced maltose syrup, xylitol, citric acid hydrate, sodium citrate hydrate, glycerin, succinic acid, sodium succinate, acetic acid, saccharin, tartaric acid, sodium tartrate, sucralose, thaumatin, sodium bicarbonate, capsicum, trehalose, white soft sugar, honey, povidone, D-mannitol, and menthol.

A wetting agent prevents the edible film 10 from drying and increases the flexibility of the edible film 10. Examples of wetting agent materials include reduced syrup, glycerin, sucrose fatty acid esters, D-sorbitol, propylene glycol, polysorbate, macrogol, and methylcellulose.

A coloring agent colors the edible film 10. Examples of coloring agent materials include titanium dioxide, food dyes, and talc.

An emulsifier facilitates mixing of ingredients. Examples of emulsifier materials include polysorbate, purified soybean lecithin, medium-chain triglycerides, and sodium lauryl sulfate.

A method for manufacturing the edible film 10 according to an embodiment of the present disclosure which does not form part of the present invention will now be described briefly with reference to the drawings.

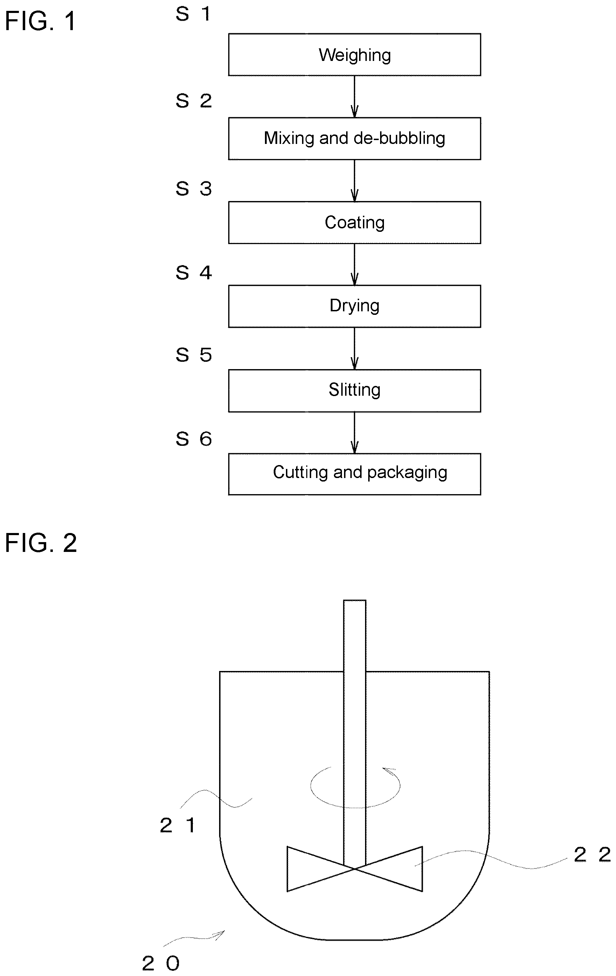

As shown in FIG. 1, a base material, ingredients, and a liquid are weighed (step S1). The base material, the ingredients, and the liquid that have been weighed are then mixed while being de-bubbled to prepare the solution 32 containing the base material and the ingredients (step S2). A carrier film 31 is coated with the solution 32 (step S3). A coating layer of the solution 32 is then dried (step S4). Slits are formed in the carrier film 31 and the layer of the solution 32 on the carrier film 31 (step S5). The layer of the solution 32 on the carrier film 31 is then cut into pieces to obtain pieces of edible film 10. Finally, the resulting pieces of edible film 10 are packaged (step S6).

Each of the steps shown in FIG. 1 will now be described in detail.

### Step 1

The weighing process will be described.

In the weighing process, the base material, the ingredients, and the liquid are weighed. The liquid may be an edible liquid. Examples of edible liquids include water, edible alcohols, edible glycols, glycerin, and edible fats. Examples of edible alcohols include ethyl alcohol. Examples of edible glycols include propylene glycol.

### Step 2

The mixing and de-bubbling process will be described.

As shown in FIG. 2, the base material, the ingredients, and the liquid that have been weighed are placed in a tank 21 of a mixer 20 with a de-bubbling capability. A blade 22 is then rotated in the tank 21 for mixing to prepare the solution 32 that is the liquid containing the base material and the ingredients. In this embodiment, a solution refers to a solution in which a base material and ingredients are dissolved in a liquid and also to a colloidal solution and a suspension containing a base material and ingredients dispersed in a liquid.

### Step 3

The coating process will be described.

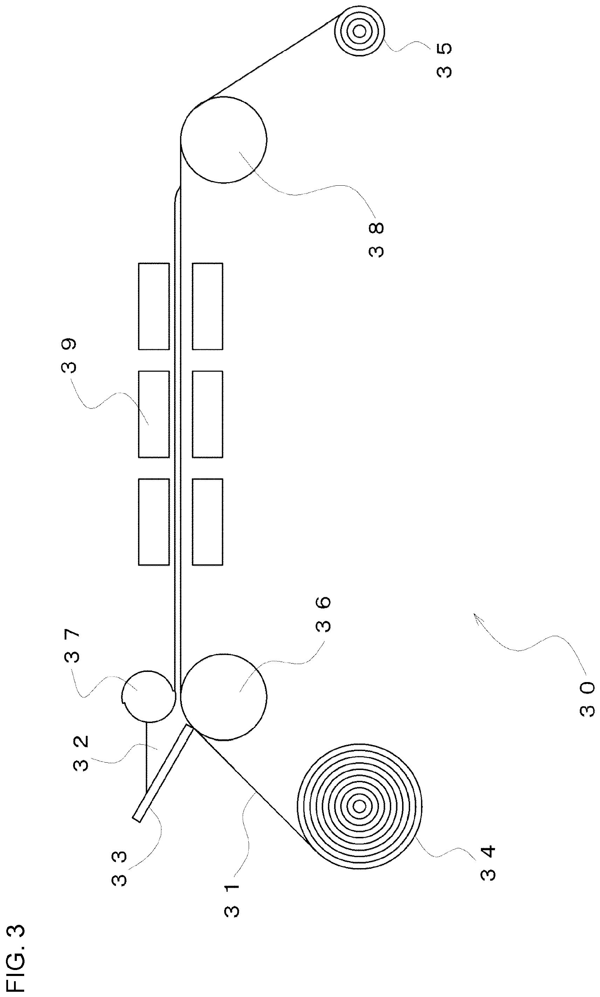

As shown in FIG. 3, the solution 32 is pooled in a liquid dam 33 in a coating device 30. While the carrier film 31 is fed from an unwinder 34 to a winder 35, the carrier film 31 is coated with the solution 32 pooled in the liquid dam 33. In the coating device 30, the thickness of the coating layer of the solution 32 is adjustable by changing the height of a coating head 37 relative to a coating roll 36 that supports the carrier film 31. The thickness of the coating layer of the solution 32 may be 10 to 1000 µm. A thickness of 10 µm or greater allows the edible film 10 after drying to be not too thin but thick enough to contain sufficient amounts of ingredients. A thickness of 1000 µm or less reduces spreading or aggregation of the solution 32 during drying of the solution 32, allowing uniform distribution of the ingredients in the resulting edible film 10. Additionally, the drying process of the solution 32 does not take long and can thoroughly dry up to the inside of the layer of the solution 32.

### Step 4

The drying process will be described.

As shown in FIG. 3, the coating layer of the solution 32 on the carrier film 31 is dried in a dryer 39 with warm air at, for example, 100 °C. The dried solution 32, together with the carrier film 31, passes over a guide roll 38 to be wound around the winder 35.

### Step 5

The slitting process will be described.

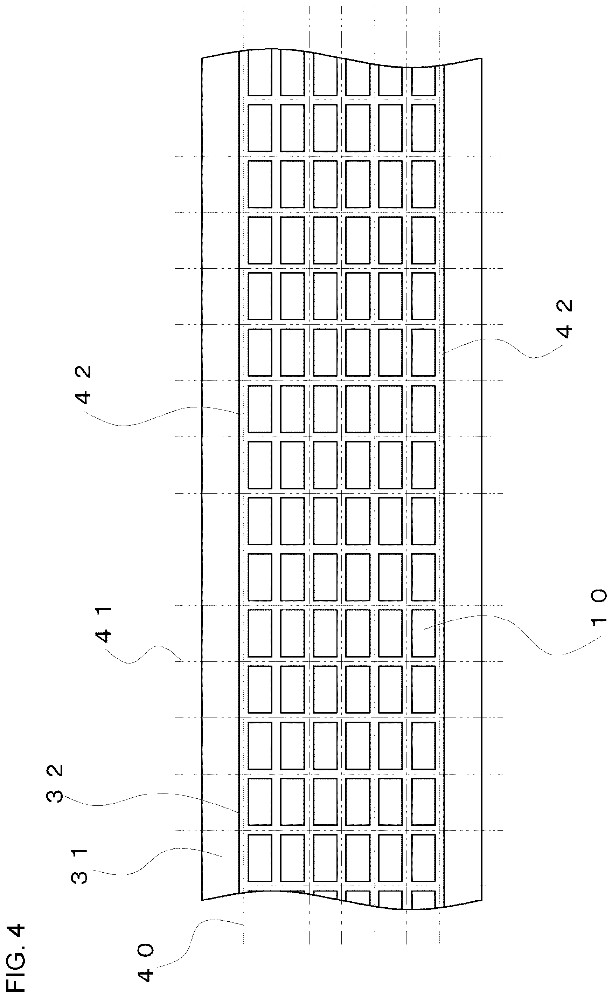

A wound-up roll of the carrier film 31 is cut into multiple rolls by a slitter machine (not shown). For example, this step may be performed in a roll-to-roll system. As shown in FIG. 4, slits 40 are formed in the carrier film 31 and the coating layer of the solution 32 on the carrier film 31 in the direction of feed of the carrier film 31 to obtain pieces of edible film 10 each with an intended size. The solution 32 in areas near ends 42 in the width direction may be raised by the coffee stain effect to be thicker than the intended thickness, and thus may or may not be used as the edible film 10.

### Step 6

The cutting and packaging process will be described.

The coating layer of the solution 32 on the carrier film 31 on each cut roll is cut into pieces by a cutting and packaging machine (not shown) to obtain pieces of edible film 10. For example, this step may be performed in a roll-to-roll system. As shown in FIG. 4, the solution 32 is cut in the width direction of the carrier film 31 along cut lines 41. This is a half-cutting process that cuts the layer of the solution 32 on the carrier film 31 alone without cutting the carrier film 31. After the layer of the solution 32 is cut into pieces along the cut lines 41, the carrier film 31 is peeled off to obtain the pieces of the edible film 10. The resulting pieces of the edible film 10 are transferred to a conveyor (not shown) to be packaged with the cutting and packaging machine. The carrier film 31 that has been peeled is rewound in a roll-to-roll system. FIG. 4 shows pieces of the edible film 10 arranged with gaps between adjacent pieces, but in the actual process, the slits 40 and the cut lines 41 may be formed without gaps between adjacent pieces of edible film 10.

The advantages and effects of using microcrystalline cellulose as one of the ingredients will now be described. Edible films in examples 1 to 3 and comparative examples 1 to 6 were prepared as shown in Table 1, wherein examples 1 to 3 and comparative examples 1 to 6 are all not according to the present invention. Although Table 1 shows the contents of the base materials and microcrystalline cellulose, the edible films also contained small amounts of additives. Maltitol, acesulfame potassium, and lactose were used as additives. In example 3 and comparative examples 5 and 6, corn starch was further used. In Table 1, the base material contents in the solution and the microcrystalline cellulose contents in the solution are each shown as weight percent of the base material and the microcrystalline cellulose dissolved in a water-ethanol mixture solution. The base material content in the solid content and the microcrystalline cellulose content in the solid content are shown as weight percent of the base material or the microcrystalline cellulose contained in the edible film obtained by drying the solution. Viscosity η₀ is the zero shear viscosity at a temperature of 25 °C. Viscosity η₁₀₀ is the viscosity of the solution measured at a temperature of 25 °C and a shear rate of 100/s. Microcrystalline cellulose was contained as an ingredient in examples 1 to 3, but not in comparative examples 1 to 6.

**Table 1**

| | Base material | Base material content in solution (wt%) | Microcrystalline cellulose content in solution (wt%) | Base material content in solid content (wt%) | Microcrystalline cellulose content in solid content (wt%) | Viscosity η₀ (Pa·s) | Viscosity η₁₀₀ (Pa·s) | η₀/η₁₀₀ |
|---|---|---|---|---|---|---|---|---|
| Example 1 | PVA | 14.5 | 20.0 | 29.0 | 40.0 | 116.9 | 41.3 | 2.8 |
| Example 2 | PEO | 15.8 | 18.0 | 48.0 | 40.0 | 87.2 | 35.2 | 2.5 |
| Example 3 | PVP | 22.8 | 26.0 | 48.0 | 40.0 | 71.0 | 23.7 | 3.0 |
| Comparative example 1 | PVA | 24.0 | 0 | 29.0 | 0 | 274.4 | 74.5 | 3.7 |
| Comparative example 2 | PVA | 18.2 | 0 | 58.0 | 0 | 13.7 | 9.3 | 1.5 |
| Comparative example 3 | PEO | 26.1 | 0 | 58.0 | 0 | 102.7 | 36.9 | 2.8 |
| Comparative example 4 | PEO | 19.1 | 0 | 58.0 | 0 | 17.5 | 10.7 | 1.7 |
| Comparative example 5 | PVP | 37.7 | 0 | 58.0 | 0 | 35.8 | 33.3 | 1.1 |
| Comparative example 6 | PVP | 30.7 | 0 | 58.0 | 0 | 1.0 | 1.0 | 1.1 |

The viscosity of the solution was measured at a temperature of 25 °C over a range of shear rates from 10 to 200/s with the continuous ramp method, using a rotational rheometer (AR-G2 manufactured by TA Instruments, Ltd.) and a steel parallel plate with a diameter of 40 mm. The viscosity data in the shear rate range of 10 to 20/s was approximated linearly by the least-squares method, and extrapolated to the zero shear rate point to estimate the zero shear viscosity η₀.

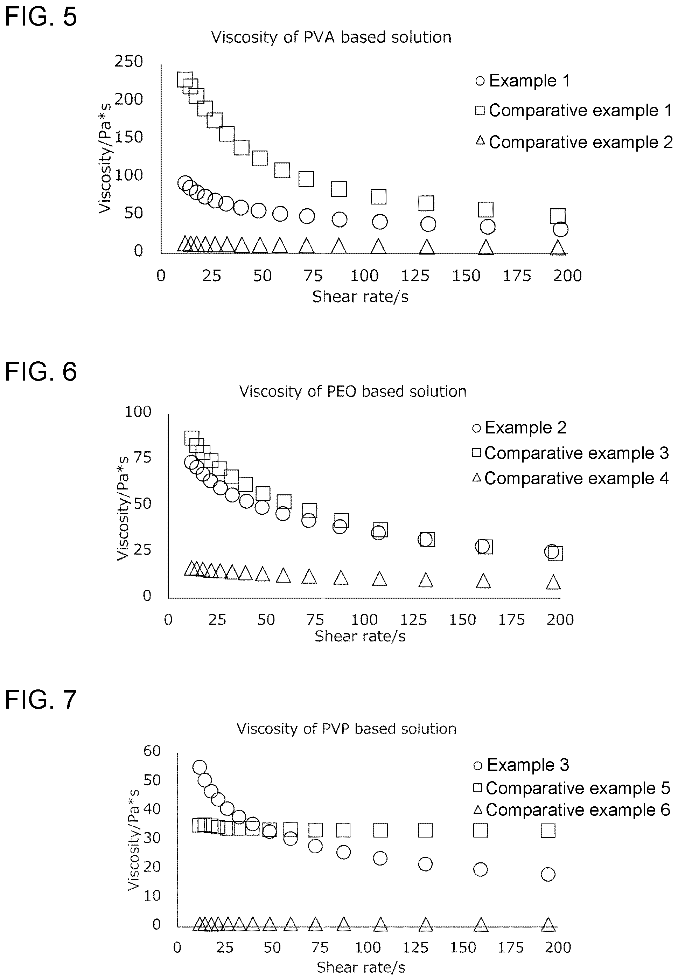

FIG. 5 shows the relationship between shear rate and viscosity measured with the solutions in example 1, comparative example 1, and comparative example 2, each containing PVA as the base material. In a solution with high thixotropy, viscosity is higher at lower shear rates, and viscosity is lower at higher shear rates. A solution with high thixotropy facilitates the coating process and reduces convection in the solution during the drying process after coating, allowing uniform distribution of ingredients in the resulting edible film. Thixotropy is evaluated using η₀/η₁₀₀, or in other words, the ratio of the viscosity η₀ to the viscosity η₁₀₀. A greater η₀/η₁₀₀ value indicates higher thixotropy. Thus, for the η₀/η₁₀₀ value to be greater, the curve showing the relationship between the shear rate and the viscosity may decline more sharply rightward, and η₀/η₁₀₀ may be 1.5 to 10, or more specifically, 2 to 10.

Typically, a solution with a higher base material content has higher thixotropy. However, higher base material content in the solution decreases the solubility and disintegratability of the edible film obtained by drying the solution, causing slower release of the ingredients from the edible film. Thus, thixotropy of the solution is to be improved at lower base material content levels.

The base material content in the solution was 14.5 wt% in example 1, 24.0 wt% in comparative example 1, and 18.2 wt% in comparative example 2. The solution in example 1 had a base material content lower than the solutions in comparative examples 1 and 2, but had high thixotropy with η₀/η₁₀₀ of 2.83. Comparative example 1 had the highest thixotropy with η₀/η₁₀₀ of 3.68, but had a high base material content of 24.0 wt% in the solution, possibly causing poor solubility and disintegratability in the resulting edible film. In comparative example 2, the curve showing the relationship between the shear rate and the viscosity does not decline rightward, but is substantially horizontal, with η₀/η₁₀₀ being 1.48 indicating low thixotropy.

FIG. 6 shows the relationship between shear rate and viscosity measured with the solutions in example 2, comparative example 3, and comparative example 4, each containing PEO as the base material. The base material content in the solution was 15.8 wt% in example 2, 26.1 wt% in comparative example 3, and 19.1 wt% in comparative example 4. The solution in example 2 had a base material content lower than the solutions in comparative examples 3 and 4, but showed high thixotropy with η₀/η₁₀₀ of 2.48, which was substantially equal to η₀/η₁₀₀ of 2.78 in comparative example 3 in which the PEO content was the highest. Comparative example 4 had η₀/η₁₀₀ of 1.65, which was within the range of high thixotropy, but the thixotropy was far lower than the thixotropy in example 2 in which the PEO content was less than the PEO content in comparative example 4.

FIG. 7 shows the relationship between shear rate and viscosity measured with solutions in example 3, comparative example 5, and comparative example 6, each containing PVP as the base material. The base material content in the solution was 22.8 wt% in example 3, 37.7 wt% in comparative example 5, and 30.7 wt% in comparative example 6. The solution in example 3 had a base material content lower than the solutions in comparative examples 5 and 6, but had high thixotropy with η₀/η₁₀₀ of 3.00. In comparative examples 5 and 6, the curves showing the relationship between the shear rate and the viscosity do not decline rightward, but are substantially horizontal, with η₀/η₁₀₀ being 1.07 in comparative example 5 and 1.06 in comparative example 6 indicating low thixotropy.

As described above, the use of microcrystalline cellulose as an ingredient improves thixotropy while reducing the base material content. When an edible film is manufactured by preparing a solution containing microcrystalline cellulose with η₀/η₁₀₀ of 1.5 to 10, or more specifically 2 to 10, the high thixotropy of the solution allows the coating process using the solution to be easier and allows the edible film obtained by drying after coating to have uniform distribution of ingredients.

The advantages and effects of using maltitol as one of the ingredients will now be described.

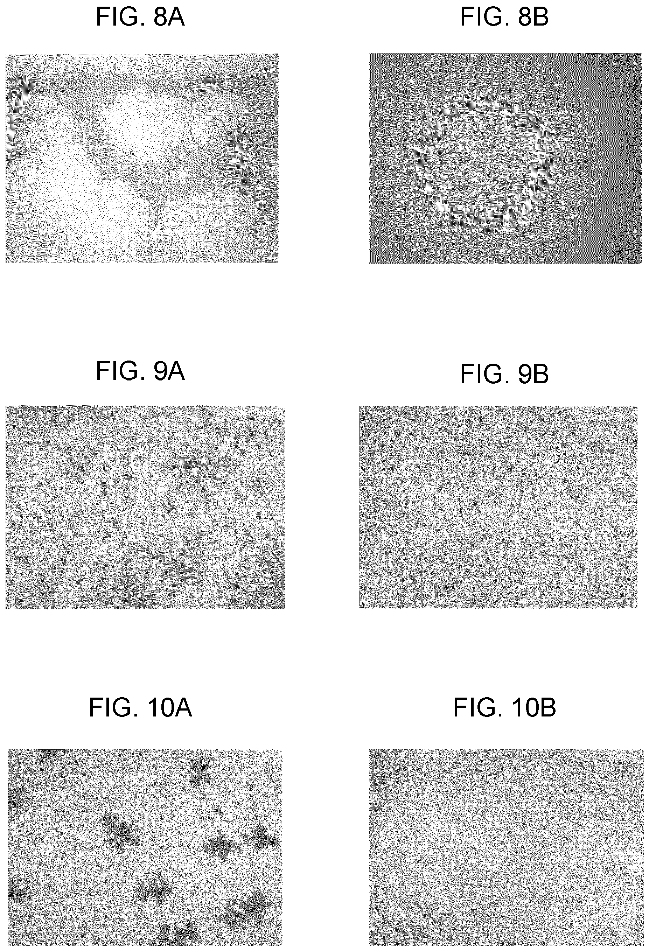

FIG. 8A is a micrograph of an edible film containing PVA as the base material and not containing maltitol. FIG. 8B is a micrograph of an edible film containing PVA as the base material and containing maltitol. FIG. 8A shows aggregated precipitation of an ingredient in the edible film not containing maltitol. The aggregated precipitation is microcrystalline cellulose. FIG. 8B shows the edible film containing maltitol with uniform distribution of ingredients without aggregation.

FIG. 9A is a micrograph of an edible film containing PEO as the base material and not containing maltitol. FIG. 9B is a micrograph of an edible film containing PEO as the base material and containing maltitol. FIG. 10A is a micrograph of an edible film containing PVP as the base material and not containing maltitol. FIG. 10B is a micrograph of an edible film containing PVP as the base material and containing maltitol. Similarly to the edible films using PVA as the base material, the edible films shown in FIGS. 9A and 10A using PEO or PVP as the base material but not containing maltitol had aggregated precipitation, whereas the edible films shown in FIGS. 9B and 10B containing maltitol had the ingredients distributed uniformly without aggregation.

As described above, maltitol reduces aggregation of microcrystalline cellulose.

The optimum maltitol content for each type of base material will now be described. The maltitol content is determined based on the microcrystalline cellulose content to have the ratio of the microcrystalline cellulose content to the maltitol content within the optimal range. In this embodiment, the microcrystalline cellulose content and the maltitol content refer to the amounts of microcrystalline cellulose and maltitol contained in the edible film in a solid state obtained by drying the solution.

Edible films using PVA as the base material were prepared by adjusting the ratio of microcrystalline cellulose content to maltitol content (the ratio of microcrystalline cellulose to maltitol), as shown in Table 2 (examples 4 to 7 and comparative examples 7 to 9). In the examples, the microcrystalline cellulose content and the maltitol content refer respectively to the amount of microcrystalline cellulose and the amount of maltitol contained in the edible film in a solid state obtained by drying the solution. Observations were performed with a microscope on examples 4 to 7 and comparative examples 7 to 9 for any ingredient aggregation. The results showed that no ingredient aggregation was observed in examples 4 to 7 in which the ratio of microcrystalline cellulose to maltitol was within a range of 0.5 and 3.3. In contrast, ingredient aggregation was observed in comparative examples 7 to 9 in which the ratio of microcrystalline cellulose to maltitol was outside the range of 0.5 and 3.3.

As described above, a solution containing PVA as the base material is unlikely to have ingredient aggregation at a ratio of microcrystalline cellulose to maltitol between 0.5 and 3.3 when the total content of the base material (PVA) and all other ingredients in the edible film is 100 wt%, allowing uniform distribution of ingredients in the resulting edible film.

**Table 2**

| | PVA (wt%) | Microcrystalline cellulose (wt%) | Maltitol (wt%) | Corn starch (wt%) | Citric acid (wt%) | Sodium citrate (wt%) | Acesulfame potassium (wt%) | Propylene glycol (wt%) | Ratio of microcrystalline cellulose to maltitol | Aggregation |
|---|---|---|---|---|---|---|---|---|---|---|
| Example 4 | 23.0 | 15.3 | 30.3 | 15.3 | 3.0 | 1.3 | 4.3 | 7.5 | 0.5 | Not observed |
| Example 5 | 23.0 | 23.0 | 15.0 | 23.0 | 3.0 | 1.3 | 4.3 | 7.5 | 1.5 | Not observed |
| Example 6 | 23.0 | 25.5 | 10.0 | 25.5 | 3.0 | 1.3 | 4.3 | 7.5 | 2.5 | Not observed |
| Example 7 | 23.0 | 26.4 | 8.1 | 26.4 | 3.0 | 1.3 | 4.3 | 7.5 | 3.3 | Observed |
| Comparative example 7 | 23.0 | 27.4 | 6.2 | 27.4 | 3.0 | 1.3 | 4.3 | 7.5 | 4.4 | Observed |
| Comparative example 8 | 23.0 | 28.3 | 4.2 | 28.3 | 3.0 | 1.3 | 4.3 | 7.5 | 6.7 | Observed |
| Comparative example 9 | 23.0 | 29.3 | 2.3 | 29.3 | 3.0 | 1.3 | 4.3 | 7.5 | 12.8 | Observed |

Edible films using PEO as the base material were prepared by adjusting the ratio of microcrystalline cellulose content to maltitol content (ratio of microcrystalline cellulose to maltitol), as shown in Table 3 (examples 8 to 11 and comparative examples 10 to 12). Observations were performed with a microscope on examples 8 to 11 and comparative examples 10 to 12 for any ingredient aggregation. The results showed that no ingredient aggregation was observed in examples 8 to 11 in which the ratio of microcrystalline cellulose to maltitol was within a range of 0.5 to 7.5. In contrast, ingredient aggregation was observed in comparative examples 10 to 12 in which the ratio of microcrystalline cellulose to maltitol was outside the range of 0.5 and 7.5.

As described above, a solution containing PEO as the base material is unlikely to have ingredient aggregation at a ratio of microcrystalline cellulose to maltitol between 0.5 and 7.5 when the total content of the base material (PEO) and all other ingredients in the edible film is 100 wt%, allowing uniform distribution of ingredients in the resulting edible film.

**Table 3**

| | PEO (wt%) | Microcrystalline cellulose (wt%) | Maltitol (wt%) | Corn starch (wt%) | Ratio of microcrystalline cellulose to maltitol | Aggregation |
|---|---|---|---|---|---|---|
| Example 8 | 50.0 | 10.0 | 20.0 | 20.0 | 0.5 | Not observed |
| Example 9 | 50.0 | 20.0 | 10.0 | 20.0 | 2.0 | Not observed |
| Example 10 | 50.0 | 25.0 | 5.0 | 20.0 | 5.0 | Not observed |
| Example 11 | 50.0 | 26.5 | 3.5 | 20.0 | 7.5 | Not observed |
| Comparative example 10 | 50.0 | 27.3 | 2.7 | 20.0 | 10.0 | Observed |
| Comparative example 11 | 50.0 | 28.0 | 2.0 | 20.0 | 14.0 | Observed |
| Comparative example 12 | 50.0 | 30.0 | 0 | 20.0 | - | Observed |

Edible films using PVP as the base material were prepared by adjusting the ratio of microcrystalline cellulose content to maltitol content (the ratio of microcrystalline cellulose to maltitol), as shown in Table 4 (examples 12 to 16 and comparative examples 13 and 14). Observations were performed with a microscope on examples 12 to 16 and comparative examples 13 and 14 for any ingredient aggregation. The results showed that no ingredient aggregation was observed in examples 12 to 16 in which the ratio of microcrystalline cellulose to maltitol was within a range from 0.67 to 7.5. In contrast, ingredient aggregation was observed in comparative examples 13 and 14 in which the ratio of microcrystalline cellulose to maltitol was outside the range of 0.67 and 7.5.

As described above, a solution containing PVP as the base material is unlikely to have ingredient aggregation at a ratio of microcrystalline cellulose to maltitol between 0.67 and 7.5

**Table 4**

| | PVP (wt%) | Microcrystalline cellulose (wt%) | Maltitol (wt%) | Corn starch (wt%) | Citric acid (wt%) | Sodium citrate (wt%) | Acesulfame potassium (wt%) | Ration of microcrystalline cellulose to Maltitol | Aggregation |
|---|---|---|---|---|---|---|---|---|---|
| Example 12 | 45.0 | 20.0 | 30.0 | 0 | 2.0 | 2.0 | 1.0 | 0.67 | Not observed |
| Example 13 | 45.0 | 20.0 | 20.0 | 10.0 | 2.0 | 2.0 | 1.0 | 1.0 | Not observed |
| Example 14 | 45.0 | 20.0 | 10.0 | 20.0 | 2.0 | 2.0 | 1.0 | 2.0 | Not observed |
| Example 15 | 45.0 | 20.0 | 5.0 | 25.0 | 2.0 | 2.0 | 1.0 | 4.0 | Not observed |
| Example 16 | 45.0 | 20.0 | 2.7 | 27.3 | 2.0 | 2.0 | 1.0 | 7.5 | Not observed |
| Comparative example 13 | 45.0 | 20.0 | 2.0 | 28.0 | 2.0 | 2.0 | 1.0 | 10.0 | Observed |
| Comparative example 14 | 45.0 | 20.0 | 0 | 30.0 | 2.0 | 2.0 | 1.0 | - | Observed |

when the total content of the base material (PVP) and all other ingredients in the edible film is 100 wt%, allowing uniform distribution of ingredients in the resulting edible film.

With the method for manufacturing the edible film according to the above embodiment, slits were formed in both the carrier film and the layer of the solution on the carrier film, but the slits may be formed in the layer of the solution as a coating alone without being formed in the carrier film.

With the method for manufacturing the edible film according to the above embodiment, the layer of the solution on the carrier film alone is cut in a half-cutting process without the carrier film being cut, but the carrier film may be cut together with the layer of the solution. In this case, the edible film may be packaged together with the carrier film without the carrier film being peeled from the pieces of edible film obtained by cutting the solution layer into pieces.

Additionally, with the method for manufacturing the edible film according to the above embodiment, slitting and cutting are separate processes, but slitting and cutting may be performed as one process in which the dried solution layer is cut into pieces.

### REFERENCE SIGNS LIST

- 10: edible film
- 31: carrier film
- 32: solution

## Claims

1. An edible film (10), comprising:
a base material; and
ingredients,
wherein the base material is one of polyvinyl alcohol, polyethylene oxide, or polyvinylpyrrolidone,
the ingredients include microcrystalline cellulose and maltitol,
a content of the microcrystalline cellulose is 5 to 50 wt%, and
wherein
the base material is polyvinyl alcohol, and
a weight ratio of a content of the microcrystalline cellulose to a content of the maltitol is 0.5 to 3.3, or wherein
the base material is polyethylene oxide, and
a weight ratio of a content of the microcrystalline cellulose to a content of the maltitol is 0.5 to 7.5, or wherein
the base material is polyvinylpyrrolidone, and
a weight ratio of a content of the microcrystalline cellulose to a content of the maltitol is 0.67 to 7.5.

## Patentansprüche

1. Essbare Folie (10), umfassend:
ein Grundmaterial; und
Inhaltsstoffe,
wobei das Grundmaterial eines von Polyvinylalkohol, Polyethylenoxid oder Polyvinylpyrrolidon ist,
die Inhaltsstoffe mikrokristalline Cellulose und Maltitol umfassen,
der Gehalt an mikrokristalliner Cellulose 5 bis 50 Gew.-% beträgt und
wobei
das Grundmaterial Polyvinylalkohol ist und
das Gewichtsverhältnis des Gehalts an mikrokristalliner Cellulose zum Gehalt an Maltitol 0,5 bis 3,3 beträgt, oder wobei
das Grundmaterial Polyethylenoxid ist und
ein Gewichtsverhältnis des Gehalts an mikrokristalliner Cellulose zum Gehalt an Maltitol 0,5 bis 7,5 beträgt, oder wobei
das Grundmaterial Polyvinylpyrrolidon ist und
das Gewichtsverhältnis des Gehalts an mikrokristalliner Cellulose zum Gehalt an Maltitol 0,67 bis 7,5 beträgt.

## Revendications

1. Film comestible (10), comprenant:
un matériau de base; et
des ingrédients,
dans lequel le matériau de base est l'un parmi l'alcool polyvinylique, le polyéthylène oxyde ou la polyvinylpyrrolidone,
les ingrédients comprennent de la cellulose microcristalline et du maltitol,
la teneur en cellulose microcristalline est comprise entre 5 et 50 % en poids, et
dans lequel
le matériau de base est l'alcool polyvinylique, et
le rapport pondéral entre la teneur en cellulose microcristalline et la teneur en maltitol est compris entre 0,5 et 3,3, ou dans lequel
le matériau de base est du poly(oxyde d'éthylène), et
le rapport pondéral entre la teneur en cellulose microcristalline et la teneur en maltitol est compris entre 0,5 et 7,5, ou dans lequel
le matériau de base est la polyvinylpyrrolidone, et
le rapport pondéral entre la teneur en cellulose microcristalline et la teneur en maltitol est compris entre 0,67 et 7,5.
